# EUROPÄISCHE PATENTSCHRIFT

(11) **EP 3 560 693 B1**
(45) Veröffentlichungstag und Bekanntmachung des Hinweises auf die Patenterteilung: **22.12.2021**
(21) Anmeldenummer: 19171272.8
(22) Anmeldetag: 26.04.2019
(51) Int. Cl.: B29C 70/44, B29C 70/54

(54) **ANSCHLUSSADAPTER ZUM VERBINDEN EINER FLUIDLEITUNG EINER VORRICHTUNG ZUR VAKUUMINFUSION**
CONNECTION ADAPTER FOR CONNECTING A FLUID LINE OF A DEVICE FOR VACUUM INFUSION
ADAPTATEUR DE RACCORDEMENT PERMETTANT DE RACCORDER UNE CONDUITE DE FLUIDE À UN DISPOSITIF D'INFUSION SOUS VIDE

(30) Priorität: 27.04.2018 DE 102018110162
(43) Veröffentlichungstag der Anmeldung: 30.10.2019
(73) Patentinhaber: Airbus Operations GmbH, 21129 Hamburg (DE)
(72) Erfinder: Mayer, Marc, 21129 Hamburg (DE); Hinz, Remo, 21129 Hamburg (DE)

(56) Entgegenhaltungen:
- EP-A1- 2 343 181
- GB-A- 2 528 851
- US-A1- 2007 132 142

## Beschreibung

### TECHNISCHES GEBIET

Die Erfindung betrifft einen Anschlussadapter zum Verbinden einer inneren Fluidleitung einer Vorrichtung zur Vakuuminfusion, sowie ein System zur Durchführung eines Vakuuminfusionsverfahrens.

### HINTERGRUND DER ERFINDUNG

Für die Herstellung von Bauteilen aus faserverstärkten Kunststoffen sind verschiedene Verfahren bekannt. Die Auswahl eines geeigneten Verfahrens begründet sich oft in dem Anwendungszweck und einer geplanten Anzahl von herzustellenden Bauteilen. Größere Stückzahlen und größere Bauteilabmessungen werden oft durch Vakuuminfusionsverfahren bewältigt, bei dem Faserhalbzeuge in eine Form eingebracht, von einer gasdichten Folie abgedeckt und anschließend durch Anlegen eines Unterdrucks mit Harz aus einer unter der Folie befindlichen Harzleitung beaufschlagt werden. Zum Versorgen dieser inneren Harzleitung mit einem ausreichenden Strom an Harz ist das Herstellen einer Verbindung zwischen der inneren Harzleitung und einer äußeren Harzleitung unabdingbar. Da die innere Harzleitung üblicherweise direkt in oder an dem Faserhalbzeug platziert ist, kann das Herstellen der Verbindung mühsam sein.

Es ist bekannt, Schlauchleitungen oder Ähnliches mit solchen inneren Harzleitungen zu verbinden, so dass die Schlauchleitungen dann aus dem Faserhalbzeug heraus unterhalb der Folie verlaufen, bis sie von dem Formwerkzeug ragen. Dadurch kann, abhängig von der Gestaltung des Faserhalbzeugs, später ein Abdruck der Schlauchleitung auf dem hergestellten Bauteil entstehen.

Beispielsweise zeigt DE 10 013 409 C1 ein Verfahren zur Herstellung von faserverstärkten Kunststoff-Bauteilen aus trockenen Faserverbund-Halbzeugen mittels eines Injektionsverfahrens, bei dem derartige Schlauchleitungen zum Versorgen der Faserhalbzeuge mit Harz verwendet werden.

Die GB2528851 zeigt eine Harzeinlassvorrichtung mit einer Harzeinlassöffnung die auf einem Harzübertragungskanal aufsetzbar ist. Die auf dem Harzübertragungskanal aufgesetzte Harzeinlassöffnung wird während der Herstellung einer Faserverbundstruktur vollständig durch eine Vakuumfolie überdeckt.

Die US2007132142 offenbart eine wiederverwendbare Öffnungsvorrichtung, die in einem Vakuum-Infusionsverfahren genutzt werden kann, um einen Vakuumaufbau mit einem Vakuum zu beaufschlagen. Dabei wird eine Vakuumfolie mittels eines Ringes mit einem O-Ring in die Öffnungsvorrichtung gedrückt, so dass ein Verrutschen der Folie verhindert werden kann.

EP2343181 zeigt eine Basis, die während der Herstellung eines Bauteils auf eine Faserpackung gelegt und mit einer Vakuumfolie abgedeckt wird. Die Vakuumfolie wird mittels einer Armatur und eines Klemmringes mit der Basis verklemmt, so dass ein Vakuumaufbau mit einem Vakuum beaufschlagt werden kann.

### ZUSAMMENFASSUNG DER ERFINDUNG

Es kann als eine Aufgabe angesehen werden, bekannte Verfahren dahingehend zu verbessern, dass das Anschließen einer Fluidleitung an eine externe Fluidquelle, z.B. eine Harzleitung, oder Fluidsenke, z.B. eine Vakuumpumpe, durchführbar ist, ohne Abdrücke oder andere nachzubessernde Stellen an dem herzustellenden Bauteil zu verursachen.

Diese Aufgabe wird durch einen Anschlussadapter mit den Merkmalen des unabhängigen Anspruchs 1 gelöst. Vorteilhafte Ausführungsformen und Weiterbildungen sind den Unteransprüchen und der nachfolgenden Beschreibung zu entnehmen.

Es wird ein Anschlussadapter zum Verbinden einer inneren Fluidleitung einer Vorrichtung zur Vakuuminfusion vorgeschlagen, wobei der Anschlussadapter eine Auflageseite und eine Anschlussseite aufweist, wobei die Auflageseite zwei Auflageschenkel zum Auflegen auf eine Fluidleitung aufweist, die einen Zwischenraum ausbilden, der sich in einer von der Anschlussseite abgewandten Richtung nach außen aufweitet, und wobei die Anschlussseite einen hohlzylindrischen Abschnitt mit einer von der Auflageseite abgewandten Anschlussöffnung und eine in die Auflageseite ragende Verbindungsöffnung und eine Fluidverbindung zwischen der Anschlussöffnung und der Verbindungsöffnung aufweist. Der hohlzylindrische Abschnitt weist einen ovalen Querschnitt auf.

Durch den Anschlussadapter wird eine Reihe von Vorteilen erreicht. Es ist zum Einen nicht mehr erforderlich, eine separate Schlauchleitung unterhalb einer Vakuumfolie zu verlegen, so dass hierdurch eine etwaige geometrische Beeinträchtigung eines herzustellenden Bauteils verhindert wird. Zum Anderen ist es möglich, praktisch an beliebigen Stellen auf dem Formwerkzeug an gewünschten Stellen jeder inneren Fluidleitung einen solchen Anschlussadapter anzubringen und durch (vorheriges) Bohren eines Lochs in die innere Leitung daran anzuschließen. Es sei darauf hingewiesen, dass ein erfindungsgemäßer Anschlussadapter nicht nur zum Führen von Harz von außen in eine innere Harzleitung als Fluidleitung durchführbar ist. Der Anschlussadapter kann auch zum Absaugen von Luft aus dem Formwerkzeug verwendet werden, wenn er an eine innere Vakuumleitung als Fluidleitung angeschlossen wird.

Das Führen von Harz oder Luft geschieht einfach durch Auflegen des Anschlussadapters und Verbinden mit der inneren Fluidleitung durch (vorheriges) Anfertigen eines Lochs, so dass Harz von oben durch den Anschlussadapter in die innere Fluidleitung fließt oder Luft direkt von oberhalb des Formwerkzeugs aus den entsprechenden Fluidleitungen absaugbar ist. Folglich ist nirgends eine Zufuhrleitung unterhalb der Folie angeordnet.

Der Anschlussadapter weist für diese Funktion eine Auflageseite und eine Anschlussseite auf. Die Auflageseite ist die Seite, die der inneren Fluidleitung zugewandt ist, d. h. die Seite, welche auf der inneren Fluidleitung aufliegt. Die Anschlussseite ist die Seite, die beispielsweise mit einem Schlauch oder einer anderen Einrichtung verbindbar ist, um das Harz durch den Anschlussadapter in die innere Harzleitung hinein oder die Luft aus der inneren Vakuumleitung heraus zu führen. Die Anschlussöffnung mündet in einer Oberseite des Anschlussadapters und kann dadurch von oberhalb der Folie zum Anschließen genutzt werden.

Die innere Fluidleitung und insbesondere die innere Harzleitung ist oftmals an einer Oberseite des Faserhalbzeugs angeordnet, so dass sie sich durch die Folie abzeichnet und daher sehr leicht mit einem oder mehreren Anschlussadaptern versehen werden kann. Für die Verwendung des erfindungsgemäßen Anschlussadapters empfiehlt es sich folglich, die inneren Fluidleitungen stets an Oberseiten des Faserhalbzeugs anzuordnen. Die Auflageseite des Anschlussadapters weist daher zwei Auflageschenkel auf, die im Wesentlichen eine V-Form bilden. Diese weitet sich nach außen hin, d. h. in eine von der Anschlussseite abgewandte Richtung, auf. Der Anschlussadapter kann folglich wie ein Sattel auf eine innere Fluidleitung aufgelegt werden.

Die Anschlussseite mit dem daran angeordneten hohlzylindrischen Abschnitt dient dazu, eine notwendige Schlauchleitung mit dem Anschlussadapter zu verbinden. Durch die hohlzylindrische Erstreckung kann der Schlauch folglich um eine gewünschte Einstecktiefe in den Anschlussadapter eingesteckt werden, um die gewünschte Fluidverbindung herzustellen. Die Anschlusssseite sollte derart ausgebildet sein, dass sich die Folie harmonisch daran anschmiegt. Bevorzugt ist der hohlzylindrische Abschnitt daher an allen Kanten abgerundet und möglichst flach.

Da eine innere Fluidleitung auch mit mehreren Anschlussadaptern verbindbar ist, ist zudem der realisierbare Volumenstrom des entsprechenden Fluids in der inneren Fluidleitung gut beeinflussbar. Je mehr Anschlussadapter verwendet werden, desto höher kann der Volumenstrom in die Fluidleitung hinein oder daraus heraus sein.

Insgesamt kann der Anschlussadapter daher besonders die Harzzufuhr in eine innere Harzleitung oder das Einbringen eines Vakuums in ein Formwerkzeug eines Harzinfusionssystems verbessern, ohne auf aufwändige Leitungssysteme setzen zu müssen, die sich unterhalb der Vakuumfolie über bzw. auf Bauteilkanten erstrecken. Zudem lässt sich der Volumenstrom an Harz, der in die innere Harzleitung(en) fließt, oder von Luft, die aus inneren Vakuumleitungen nach außen fließt, sehr leicht steuern.

Ferner weist der hohlzylindrische Abschnitt einen ovalen Querschnitt auf. Der Querschnitt ist hierbei parallel zu der Schiene bzw. zu einer Ebene gesehen, die durch Endkanten der Auflageschenkel aufgespannt wird. Hierdurch wird die Ausrichtung der darauf befindlichen Folie gesteuert, da sie sich bevorzugt entlang der längeren Erstreckung des hohlzylindrischen Abschnitts faltet. Damit kann ein übermäßiger Faltenwurf vermieden werden und die Folie passt sich bei Evakuierung harmonisch an die Oberfläche des Anschlussadapters an.

In einer vorteilhaften Ausführungsform verjüngt sich mindestens ein Maß einer Ausdehnung des Querschnitts in eine von der Auflageseite abgewandte Richtung bis zu einer Oberseite. Die Folie schmiegt sich auch entlang von Seitenflächen des hohlzylindrischen Abschnitts besonders glatt an diesen an. Der Querschnitt wird wie vorangehend erwähnt bestimmt. In diesem Sinne kann ein Maß die Breite oder die Tiefe des Querschnitts sein. Den geringsten Querschnitt weist folglich eine Oberseite des hohlzylindrischen Abschnitts auf. Die Folie kann sich dabei von einem Auflagebereich auf den Schenkeln sanft über den hohlzylindrischen Abschnitt nach oben erstrecken und ein Faltenwurf oder dergleichen an den Seitenflächen wird aufgrund der leicht kegligen Form weitgehend vermieden.

In einer besonders bevorzugten Ausführungsform ist in der Anschlussöffnung ein Absatz angeordnet, um eine Einstecktiefe einer externen Fluidleitung zu begrenzen. Ein Benutzer kann folglich die externe Fluidleitung in die Anschlussöffnung einstecken, bis ein Absatz spürbar das Weiterschieben verhindert. Damit kann nicht nur garantiert werden, dass eine notwendige Einstecktiefe erreicht ist, sondern auch, dass eine Beschädigung des Faserhalbzeugs verhindert wird.

In einer vorteilhaften Ausführungsform sind die Schenkel des Anschlussadapters als flächenhafte Körper ausgeführt, die eine im Wesentlichen konstante Länge aufweisen. Der Anschlussadapter kann damit sehr gut bündig an die innere Fluidleitung angeschmiegt werden, die insbesondere als Harzleitung oftmals eine grob dreiecksförmige, weitgehend konstante äußere Kontur aufweist.

Der Anschlussadapter ist bevorzugt aus einem Kunststoff hergestellt. Hierbei ist darauf zu achten, dass der Werkstoff eine ausreichende Temperaturfestigkeit besitzt. Die Aushärtung der Bauteile, für die das Vakuuminfusionsverfahren genutzt wird, kann bei einer Temperatur von 140 bis 180 Grad durchgeführt werden. Da der Anschlussadapter nach der Evakuierung auf der Form und unterhalb der Vakuumfolie verbleibt, bis der Aushärteprozess beendet ist, sollte er daher eine entsprechende Temperaturfestigkeit besitzen.

Besonders bevorzugt ist der Anschlussadapter aus einem zumindest gummiartigen Material hergestellt. Durch die Vakuumfolie ergibt sich ein gewisser Anpressdruck auf den Anschlussadapter, so dass sich dieser auf die innere Fluidleitung drückt, um die darin befindliche Bohrung bzw. den Ausschnitt randseitig abzudichten. Ein gummiartiges Material kann deutlich besser für diese Aufgabe geeignet sein, als ein starres und formstabiles Material.

Der Anschlussadapter kann durch ein Gussverfahren hergestellt sein. Bei der Verwendung eines gummiartigen Materials kann ein gießfähiger Werkstoff auf Basis eines Zwei-Komponenten-Systems realisiert werden. Wie vorangehend erwähnt sollte darauf geachtet werden, dass der Werkstoff bei einer Temperatur von mindestens 180°C temperaturstabil ist. Es wäre vorteilhaft, einen Werkstoff auszuwählen, der bei einer Temperatur von mindestens 200°C temperaturstabil ist. Die Erfindung betrifft ferner ein System zum Herstellen eines Bauteils aus einem Faserverbundwerkstoff, aufweisend ein Formwerkzeug mit einer Auflagefläche, mindestens eine innere Fluidleitung, mindestens einen Anschlussadapter je innerer Fluidleitung und eine Vakuumfolie zum Abdecken eines Halbzeugs und eine Vakuumpumpe.

Selbstverständlich sind weitere Komponenten denkbar, die zur optimierten Ausführung eines mit dem System ausgeführten Verfahrens notwendig sind. Mindestens eine der inneren Fluidleitungen kann dabei eine innere Harzleitung sein. Alternativ oder zusätzlich dazu kann mindestens eine der inneren Fluidleitungen eine innere Vakuumleitung sein.

In einer vorteilhaften Ausführungsform weist die innere Fluidleitung einen Omega-förmigen Profilquerschnitt auf. Eine solche Ausführung einer Fluidleitung ist insbesondere bei Harzleitungen sehr weit verbreitet und kann dabei die Verteilung des einfließenden Harzes positiv beeinflussen.

### KURZE BESCHREIBUNG DER FIGUREN

Weitere Merkmale, Vorteile und Anwendungsmöglichkeiten der vorliegenden Erfindung ergeben sich aus der nachfolgenden Beschreibung der Ausführungsbeispiele und den Figuren.

In den Figuren stehen weiterhin gleiche Bezugszeichen für gleiche oder ähnliche Objekte.
Fig. 1 zeigt eine innere Harzleitung mit einem darauf angeordneten Anschlussadapter.
Fig. 2 zeigt eine innere Harzleitung mit einer Bohrung für eine Fluidverbindung zu einem Anschlussadapter.
Fig. 3 zeigt einen Anschlussadapter in einer schrägen Ansicht von oben.
Fig. 4 zeigt einen Anschlussadapter von der Seite.
Fig. 5 zeigt ein System zum Herstellen eines Bauteils aus einem Faserverbundwerkstoff in einer schematischen Darstellung.

### DETAILLIERTE DARSTELLUNG EXEMPLARISCHER

### AUSFÜHRUNGSFORMEN

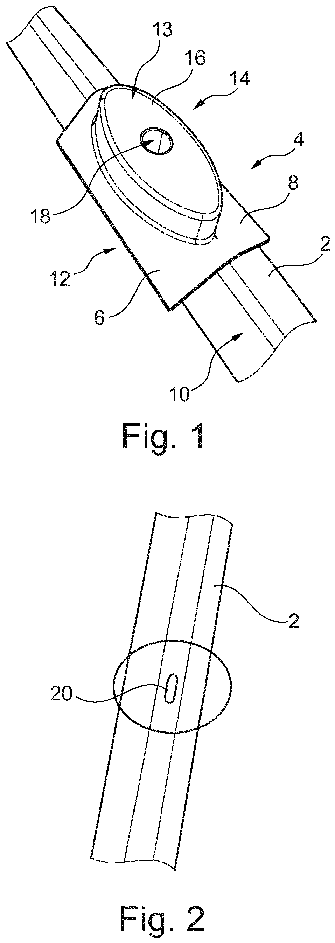

Fig. 1 zeigt eine innere Fluidleitung 2, die exemplarisch eine innere Harzleitung zum Leiten von Harz ist, für ein Formwerkzeug, sowie einen darauf aufgesetzten Anschlussadapter 4. Dieser besitzt zwei Auflageschenkel 6 und 8, die im Wesentlichen als flächige Elemente ausgeführt sind und bündig auf einer Oberfläche 10 der inneren Fluidleitung 2 angeordnet sind. Der Anschlussadapter kann daher einen guten, bündigen Kontakt mit der inneren Fluidleitung 2 herstellen.

Das Material des Anschlussadapters 4 sollte insbesondere eine ausreichende Temperaturfestigkeit besitzen und möglichst formelastisch sein. Es bietet sich an, hierzu ein gießfähiges, elastisches und bevorzugt gummielastisches Material einzusetzen, das Temperaturen von über 180°C toleriert. Es kann sich anbieten, ein gießbares Hochtemperatur-Gummi einzusetzen, welches beispielsweise raumtemperaturvernetzend ist. Derartige Materialien sind mit einer Gebrauchstemperatur von deutlich über 200 °C erhältlich. Durch ein gummiartiges Material kann zudem die Fluidverbindung zwischen der inneren Fluidleitung 2 und dem Anschlussadapter 4 verbessert werden, da eine Folie bei der Evakuierung den Anschlussadapter 4 auf die Leitung 2 presst und dabei entlang der Kontaktflächen abdichtet.

Der Anschlussadapter 4 weist neben einer Auflageseite 12, die die beiden Auflageschenkel 6 und 8 enthält, auch eine Anschlussseite 14 auf, welche einen hohlzylindrischen Abschnitt 16 besitzt. Dieser ist exemplarisch mit einem ovalen bzw. elliptischen Querschnitt ausgestattet.

Von einer Schnittkante zwischen den Auflageschenkeln 6 und 8 und dem hohlzylindrischen Abschnitt 16 erstreckt sich dieser in eine von der Auflageseite 12 abgewandte Richtung. Dabei verjüngt sich der Querschnitt bis zu einer Oberseite 13 exemplarisch leicht keglig.

Der hohlzylindrische Abschnitt 16 weist eine Anschlussöffnung 18 auf, welche sich vollständig durch den hohlzylindrischen Abschnitt 16 bis durch die Auflageschenkel 6 und 8 erstreckt und in einer Verbindungsöffnung 11 mündet. Die Anschlussöffnung 18 dient der Aufnahme einer geeigneten externen Fluidleitung, durch die in diesem Beispiel Harz in die innere Fluidleitung 2 geleitet wird. Hierfür wird, wie weiter nachfolgend in Fig. 2 gezeigt, die innere Fluidleitung 2 mit einem Ausschnitt 20 versehen. Dieser kann separat in einer inneren Fluidleitung 2 bedarfsweise hergestellt werden oder die innere Fluidleitung 2 kann bereits bei ihrer Herstellung mit mehreren, an geeigneten Stellen angeordneten Ausschnitten 20 versehen werden.

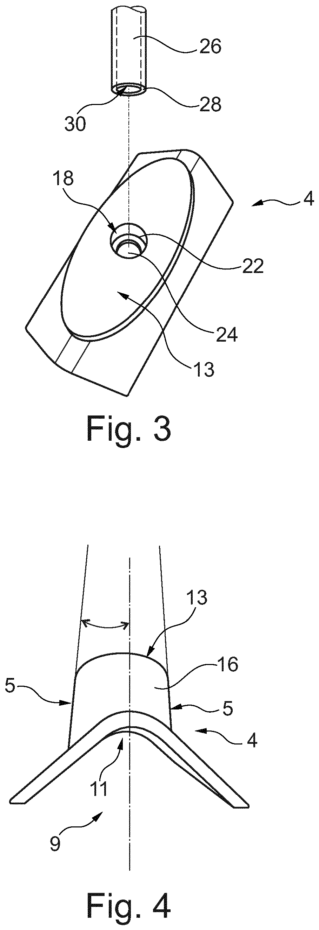

In Fig. 3 wird der Anschlussadapter 4 in einer freigestellten, dreidimensionalen Darstellung von oben gezeigt. Hier ist ein Detail der Anschlussöffnung 18 ersichtlich, die sich in das Innere des Anschlussadapters 4 erstreckt. Im Innern der Anschlussöffnung 18 in einem vorbestimmten Abstand zu der Oberseite des hohlzylindrischen Abschnitts 16 ist ein Absatz 22 angeordnet, der eine Absatzöffnung 24 besitzt, die einen geringeren Durchmesser als die Anschlussöffnung 18 aufweist. Hierdurch kann eine externe Fluidleitung 26, die etwa als Schlauch ausgeführt ist, in die Anschlussöffnung 18 eingesteckt werden. Beim Einstecken gerät eine ringförmige Endfläche 28 der externen Fluidleitung 26 mit dem Absatz 22 in Flächenkontakt. Ein Weiterschieben der Fluidleitung 26 ist dann nicht mehr möglich. Gleichzeitig wird durch eine Öffnung 30 der externen Fluidleitung 26 und die Absatzöffnung 24 eine Fluidverbindung zwischen dem Schlauch 26 und der Absatzöffnung 24 hergestellt, so dass bei aufgelegtem Anschlussadapter 4 schließlich eine Fluidverbindung mit der inneren Fluidleitung 2 hergestellt ist.

Fig. 4 zeigt eine Seitenansicht des Anschlussadapters 4. Hier ist insbesondere die leicht keglige Form des hohlzylindrischen Abschnitts 16 erkennbar. Beispielhaft ist ein solcher Kegelwinkel vorgesehen, dass Seitenflächen 5 des hohlzylindrischen Abschnitts 16 gegenüber der Vertikalen oder einer Erstreckungsrichtung der Öffnungen 11 und 18 um einen Winkel in einem Bereich von etwas weniger als 1° bis 25° angeschrägt sind. Selbstverständlich sind auch andere Kegelwinkel möglich.

Der Winkel zwischen den beiden Auflageschenkeln 6 und 8, welche einen sich in einer von der Anschlussseite 14 abgewandten Richtung erweiternden Zwischenraum 9 einschließen, könnte grob 90° betragen. Dies ist jedoch abhängig von der Ausgestaltung der verwendeten inneren Fluidleitung 2. Wird ein gummielastisches Material für die Herstellung des Anschlussadapters 4 verwendet, ist eine sehr präzise Anpassung der Form des Anschlussadapters 4 an die innere Fluidleitung 2 nicht notwendig. An einer Schnittkante zwischen den beiden Auflageschenkeln 6 und 8 befindet sich indes eine Verbindungsöffnung 11, die in einer Fluidverbindung mit der Anschlussöffnung 18 steht. Hierdurch kann in die Anschlussöffnung 18 eingeleitetes Harz in den Ausschnitt 20 einer Schiene 2 geraten.

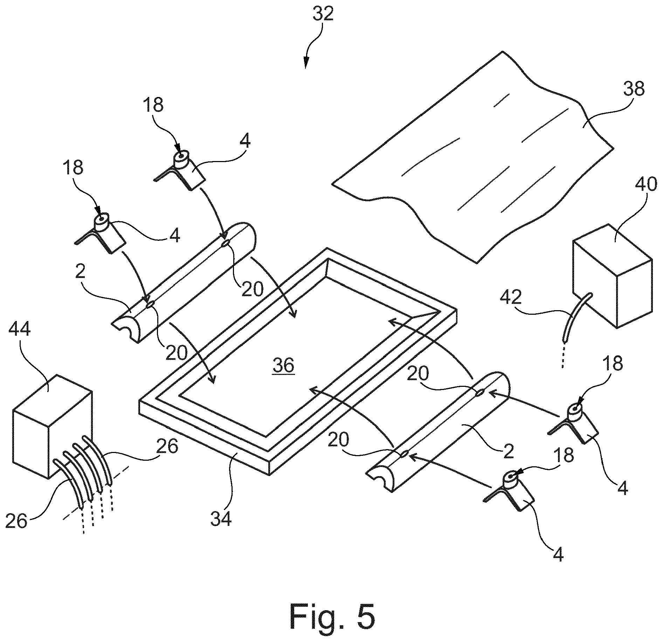

Schließlich zeigt Fig. 5 schematisch ein System 32 zum Herstellen eines Bauteils aus einem Faserverbundwerkstoff. Ein Formwerkzeug 34 dient zur Aufnahme von Faserhalbzeugen auf einer Auflagefläche 36, die die Form des Bauteils bestimmt. Zur Durchführung einer Harzinfusion ist beispielhaft eine innere Harzleitung 2 dargestellt, welche auf das Faserhalbzeug gelegt werden kann. Zusätzlich ist eine Vakuumleitung 42 vorgesehen, durch die ein Vakuum angelegt werden kann, das zur Harzinfusion führt. Zur Vorbereitung sind beispielhaft zwei Ausschnitte 20 an der inneren Harzleitung 2 vorgesehen, auf die jeweils ein Anschlussadapter aufgelegt werden kann. An der Vakuumleitung 42 sind Ausschnitte angeordnet, auf die ebenfalls Anschlussadapter 4 aufgelegt werden. Zur vakuumdichten Abdeckung ist weiterhin eine Vakuumfolie 38 vorgesehen, die auf das Faserhalbzeug und die darauf befindliche innere Fluidleitung 2 gelegt wird. Streifenförmige Dichtungen und andere Einrichtungen zum Unterstützen der Vakuuminfusion sind hier der Einfachheit halber nicht dargestellt. Zum Herstellen des Unterdrucks ist eine Vakuumpumpe 40 vorgesehen, die über eine Leitung 42 mit dem Formwerkzeug 34 bzw. der Auflagefläche 36 über die Ausschnitte und die Anschlussadapter 4 in Fluidverbindung bringbar ist.

Die erfindungsgemäßen Anschlussadapter 4 erlauben das Einleiten von Harz an mehreren Stellen einer inneren Harzleitung 2, ohne dass ein zusätzlicher Schlauch unterhalb der Vakuumfolie 38 zu verlegen ist, der auf ein herzustellendes Bauteil drückt und dessen Formtreue einschränken könnte. Zudem ist der Volumenstrom an Harz durch Wahl der geeigneten Anzahl von Anschlussadaptern 4 einstellbar. Sämtliche Anschlussadapter 4 können mit jeweils einer externen Harzleitung 26 verbunden werden, die beispielhaft als separate Leitungen aus einem Harzbehälter 44 herausgeführt werden könnten oder jeweils als Abzweigung einer größeren Harzleitung realisierbar sind. Analog gilt dies für die Anschlussadapter 4, welche mit der inneren Vakuumleitung 42 anbringbar sind.

## Patentansprüche

1. Anschlussadapter (4) zum Verbinden einer innere Fluidleitung (2, 42) einer Vorrichtung zur Vakuuminfusion,
wobei der Anschlussadapter (4) eine Auflageseite (12) und eine Anschlussseite (14) aufweist,
wobei die Auflageseite (12) zwei Auflageschenkel (6, 8) zum Auflegen auf eine Fluidleitung (2, 42) aufweist, die einen Zwischenraum (9) einschließen, der sich in einer von der Anschlussseite (14) abgewandten Richtung nach außen aufweitet,
wobei die Anschlussseite (14) einen hohlzylindrischen Abschnitt (16) mit einer von der Auflageseite (12) abgewandten Anschlussöffnung (18), und einer in die Auflageseite (12) ragende Verbindungsöffnung (11) und einer Fluidverbindung zwischen der Anschlussöffnung (18) und der Verbindungsöffnung (11) aufweist,
**dadurch gekennzeichnet, dass** der hohlzylindrische Abschnitt (16) einen ovalen Querschnitt aufweist.

2. Anschlussadapter (4) nach Anspruch 1, wobei sich mindestens ein Maß einer Ausdehnung des Querschnitts in eine von der Auflageseite (12) abgewandte Richtung bis zu einer Oberseite (13) verjüngt.

3. Anschlussadapter (4) nach Anspruch 1 oder 2, wobei in der Anschlussöffnung (18) ein Absatz (22) angeordnet ist, um eine Einstecktiefe einer externen Fluidleitung (26) zu begrenzen.

4. Anschlussadapter (4) nach einem der vorhergehenden Ansprüche, wobei die Auflageschenkel (6, 8) des Anschlussadapters (4) als flächenhafte Körper ausgeführt sind, die eine im Wesentlichen konstante Länge aufweisen.

5. Anschlussadapter (4) nach einem der vorhergehenden Ansprüche, wobei der Anschlussadapter (4) aus einem Kunststoff hergestellt ist.

6. Anschlussadapter (4) nach einem der vorhergehenden Ansprüche, wobei der Anschlussadapter (4) aus einem zumindest gummiartigen Material hergestellt ist.

7. System (32) zum Herstellen eines Bauteils aus einem Faserverbundwerkstoff, aufweisend ein Formwerkzeug (34) mit einer Auflagefläche (36), innere Fluidleitungen (2, 42), mindestens einen Anschlussadapter (4) nach einem der vorhergehenden Ansprüche für jede innere Fluidleitung (2, 42), eine Vakuumfolie (38) zum Abdecken eines Halbzeugs und eine Vakuumpumpe (40).

8. System (32) nach Anspruch 7, wobei mindestens eine der inneren Fluidleitungen (2, 42) eine innere Harzleitung ist.

9. System (32) nach Anspruch 7 oder 8, wobei mindestens eine der inneren Fluidleitungen (2, 42) eine innere Vakuumleitung ist.

10. System (32) nach einem der Ansprüche 7 bis 9, wobei die innere Fluidleitung (2, 42) einen Omega-förmigen Profilquerschnitt aufweist.

## Claims

1. Connection adapter (4) for connecting an internal fluid line (2, 42) of a vacuum infusion device,
wherein the connection adapter (4) has a support side (12) and a connection side (14),
wherein the support side (12) has two support legs (6, 8) for placing on a fluid line (2, 42), which enclose an intermediate space (9) which widens outwards in a direction facing away from the connection side (14),
wherein the connection side (14) has a hollow cylindrical section (16) with a connection opening (18) facing away from the support side (12), and a junction opening (11) projecting into the support side (12) and a fluid connection between the connection opening (18) and the junction opening (11),
**characterised in that**
the hollow cylindrical section (16) has an oval cross-section.

2. Connection adapter (4) according to claim 1, wherein at least one dimension of an extension of the cross-section tapers in a direction away from the support side (12) to an upper side (13).

3. Connection adapter (4) according to claim 1 or 2, wherein a shoulder (22) is arranged in the connection opening (18) in order to limit an insertion depth of an external fluid line (26).

4. Connection adapter (4) according to one of the preceding claims, wherein the support legs (6, 8) of the connection adapter (4) are designed as planar bodies which have a substantially constant length.

5. Connection adapter (4) according to one of the preceding claims, wherein the connection adapter (4) is made of a plastic.

6. Connection adapter (4) according to one of the preceding claims, wherein the connection adapter (4) is made of an at least rubber-like material.

7. A system (32) for producing a component from a fibre composite material, comprising a mould (34) with a support surface (36), internal fluid lines (2, 42), at least one connection adapter (4) according to one of the preceding claims for each internal fluid line (2, 42), a vacuum film (38) for covering a semi-finished product and a vacuum pump (40).

8. System (32) according to claim 7, wherein at least one of said internal fluid conduits (2, 42) is an internal resin conduit.

9. System (32) according to claim 7 or 8, wherein at least one of the internal fluid conduits (2, 42) is an inner vacuum line.

10. System (32) according to any one of claims 7 to 9, wherein the inner fluid conduit (2, 42) has an omega-shaped profile cross-section.

## Revendications

1. Adaptateur de connexion (4) pour connecter une ligne de fluide interne (2, 42) d'un dispositif de perfusion sous vide,
dans lequel l'adaptateur de connexion (4) a un côté support (12) et un côté connexion (14),
dans lequel le côté de support (12) a deux pieds de support (6, 8) à placer sur une ligne de fluide (2, 42), qui enferment un espace intermédiaire (9) qui s'élargit vers l'extérieur dans une direction opposée au côté de connexion (14),
dans lequel le côté de connexion (14) a une section cylindrique creuse (16) avec une ouverture de connexion (18) tournée à l'opposé du côté de support (12), et une ouverture de jonction (11) faisant saillie dans le côté de support (12) et une connexion de fluide entre l'ouverture de connexion (18) et l'ouverture de jonction (11),
**caractérisé en ce que**
la section cylindrique creuse (16) a une section transversale ovale.

2. Adaptateur de connexion (4) selon la revendication 1, dans lequel au moins une dimension d'une extension de la section transversale se rétrécit dans une direction s'éloignant du côté de support (12) vers un côté supérieur (13).

3. Adaptateur de connexion (4) selon la revendication 1 ou 2, dans lequel un épaulement (22) est disposé dans l'ouverture de connexion (18) afin de limiter une profondeur d'insertion d'une ligne de fluide externe (26).

4. Adaptateur de connexion (4) selon l'une des revendications précédentes, dans lequel les pieds de support (6, 8) de l'adaptateur de connexion (4) sont conçus comme des corps planaires qui ont une longueur sensiblement constante.

5. Adaptateur de connexion (4) selon l'une des revendications précédentes, dans lequel l'adaptateur de connexion (4) est constitué d'une matière plastique.

6. Adaptateur de connexion (4) selon l'une des revendications précédentes, dans lequel l'adaptateur de connexion (4) est constitué d'un matériau au moins semblable au caoutchouc.

7. Système (32) pour la fabrication d'un composant en matériau composite à fibres, comprenant un moule (34) avec une surface d'appui (36), des conduites de fluide internes (2, 42), au moins un adaptateur de connexion (4) selon l'une des revendications précédentes pour chaque conduite de fluide interne (2, 42), un film à vide (38) pour recouvrir un produit semi-fini et une pompe à vide (40).

8. Système (32) selon la revendication 7, dans lequel au moins un desdits conduits de fluide internes (2, 42) est un conduit de résine interne.

9. Système (32) selon la revendication 7 ou 8, dans lequel au moins une des conduites de fluide interne (2, 42) est une conduite de vide interne.

10. Système (32) selon l'une quelconque des revendications 7 à 9, dans lequel le conduit de fluide internes (2, 42) présente une section transversale à profil en oméga.
